# EUROPEAN PATENT SPECIFICATION

(11) **EP 1 088 554 B1**
(45) Date of publication and mention of the grant of the patent: **19.11.2003**
(21) Application number: 00121143.2
(22) Date of filing: 29.09.2000
(51) Int. Cl.: A61K 31/60, A61K 9/50, A61K 9/20

(54) **Controlled release drug association containing 5-isosorbide mononitrate and acetylsalicylic acid**
Zusammensetzung mit kontrollierter Freigabe enthaltend 5-Isosorbidnitrat und Acetylsalicylsäure
Association à libération prolongée contenant du mononitrate d'isosorbide et de l'acide acétylsalicylique

(30) Priority: 30.09.1999 BR 9905261
(43) Date of publication of application: 04.04.2001
(73) Proprietor: Libbs Farmaceutica LTDA, 05025-011 Sao Paulo (BR)
(72) Inventor: Athayde, Alcebiades de Mendonça, 05025-011 Sao Paulo (BR)
(74) Representative: Minoja, Fabrizio, Dr.

(56) References cited:
- EP-A- 0 676 204
- WO-A-98/05306
- WO-A-98/17271
- HEYDT-LIMAN A ET AL: "PHARMACOKINETICS OF ISOSORBIDE DINITRATE WHEN COADMINISTERED WITH ACETYLICSALICYLIC ACID" EUROPEAN JOURNAL OF PHARMACOLOGY,NL,AMSTERDAM, vol. 183, no. 6, 1990, page 2395 XP002054180 ISSN: 0014-2999

## Description

The present invention relates to oral pharmaceutical compositions and the process for the preparation thereof.

More specifically, the present invention relates to oral pharmaceutical compositions comprising controlled-release particles of 5-isosorbide mononitrate in combination with acetylsalicylic acid particles.

In the compositions of the invention, the acetylsalicylic acid particles may be controlled-release particles, regular-release particles, granules or coated crystals.

The compositions of the present invention are valuable in the therapy/prophylaxis of vascular cardiac diseases and/or their signs/symptoms such as, for example, angina pectoris, myocardial infarction or other vascular cardiac, cerebral or peripheral diseases.

Acetylsalicylic acid, an effective anti-inflammatory, analgesic and antipyretic agent widely used for the treatment of rheumatoid arthritis or osteoarthritis, has been used for some time also as a platelet aggregation inhibitor in patients at risk of intravascular thrombosis. Low doses of acetylsalicylic acid are effective in the reduction of the risk both of first myocardial infarction in healthy people and of reinfarction in patients who have already suffered an infarction. When there are no contraindications, its use is recommended in patients above 40 years old and who exhibit a significantly high infarction risk.

For the cardiovascular use, the recommended doses of acetylsalicylic acid vary greatly, its effectiveness reportedly varying within a range of 5 to 1500 mg a day, doses from 50 to 400 mg as a single daily administration being preferred.

Acetylsalicylic acid exerts antiplatelet action by inhibiting the cyclooxygenase enzyme through an irreversible acetylation process ("suicide" bond). The inhibition of cyclooxygenase in blood platelets prevents the synthesis of thromboxane A2, a compound with vasoconstricting action that causes platelet aggregation.

As already mentioned, inhibition of cyclooxygenase by acetylsalicylic acid takes place irreversibly and, since it is known that blood platelets can not resynthesise cyclo oxygenase, the production of platelet thromboxane A2 will be inhibited for the remaining lifespan of platelets "affected" by the acetylsalicylic acid taken. The production of thromboxane A2 will only be restored about 24 hours later, when new platelets are released by the bone marrow. This is why a single daily administration of acetylsalicylic acid is effective to obtain a platelet anti-aggregating activity.

5-Isosorbide mononitrate, an active metabolite of isosorbide dinitrate with vasodilating action, is used for the treatment and prophylaxis of angina pectoris, myocardial infarction and in the therapy of congestive heart failure. Its short plasma half-life and adverse effects due to the high doses required, make it necessary to divide the daily dose to maintain effective plasma levels. On the other hand, the need for several daily doses induces lower compliance of the patients to this treatment.

The usually recommended dose of 5-isosorbide mononitrate is 20 mg, two or three times a day, however total daily doses of up to 120 mg can be prescribed.

The drug plasma concentration should be maintained within therapeutic levels for 16 to 20 hours after the administration, since the maintenance of high plasma levels of the active compound throughout the 24 hours of the day may cause resistance to the treatment, due to the reduction of the body response to the drug.

Statistic studies on the prescription profile of pharmaceuticals indicate that, for most cases where 5-isosorbide mononitrate formulations are prescribed, a joined prescription with acetylsalicylic acid formulations is made. The joined prescription of these active compounds is based on various scientific papers that show that the administration of acetylsalicylic acid associated with nitric acid donors, a compound family which 5-isosorbide mononitrate belongs to, provides clinical advantages not only related to the increase in the drug efficacy by synergistic effect, but also to the reduction of the side and/or adverse effects thereof.

One of the problems involved in the joined prescription of formulations with isosorbide mononitrate and formulations with acetylsalicylic acid is the low compliance of the patients to the joined treatment. Studies show that the patients who suffer from angina have a good compliance to the treatment with isosorbide mononitrate alone, however, when acetylsalicylic acid is simultaneously prescribed, half the patients do not take it properly, jeopardizing the efficiency of the treatment.

The formulations that provide 5-isosorbide mononitrate and acetylsalicylic acid in a single dose unit are, therefore, valuable and advantageous for the joined therapy when compared to the current formulations of the drugs alone, since they overcome the problem of low compliance to the treatment when both drugs are prescribed.

Due to the different pharmacokinetic and pharmacodynamic profiles of acetylsalicylic acid and 5-isosorbide mononitrate, the combinations of these drugs should preferably provide controlled-release of isosorbide mononitrate, so as to allow a single daily dose of the combination.

Regular-release formulations of acetylsalicylic acid are suitable to the combination. We should not, however, rule out the use of modified-release formulations, such as enteric coating formulations, for a higher safety of the product, to reduce the damages that acetylsalicylic acid is known to exert on the gastrointestinal tract.

Drugs formulated as single tablets, particularly those with formulations with polymeric or fatty matrixes, such those of the prior art, may cause gastrointestinal damage/irritation, due to the high concentration of the drugs at the contact site of the tablet with the mucosa. This risk is even higher when the formulation includes drugs that are known to be aggressive to the gastrointestinal tract. The formulation of drugs in micro-particles disclosed in the present invention significantly reduces this risk, since it allows a better dispersion of the formulation in the digestive tract and therefore a lower concentration of the drugs at the contact sites with the mucosa.

The combinations of 5-isosorbide mononitrate and acetylsalicylic acid in a single tablet of the prior art may cause problems in patients with swallowing difficulty, due to their sizes. This drawback is avoided with the use of multiple particle formulations, such as those disclosed in the present invention, since they may be dispersed in liquids at the moment of the administration.

It should be kept in mind that pharmaceutical compositions formulated in tablets are subject to variations in their physical-chemical properties, such as hardness, disintegration time and dissolution time, due to the compression process involved in their production. Such variations are of course undesirable in slow-release isosorbide mononitrate tablets, since the prediction of the dissolution rate is an extremely important factor for the efficiency of the formulation.

The multiple particles compositions of the present invention prevent such risk, since the dissolution rate of 5-isosorbide mononitrate in these formulations is not affected by a compression process at the final step of unit doses manufacture.

Finally, the combinations of acetylsalicylic acid with controlled-release multiple particles formulations of 5-isosorbide mononitrate of the invention advantageously provide a better control of the drug release at the gastrointestinal tract, compared with single tablets formulations, in that isosorbide mononitrate multiple particles allow to prepare different types of formulations, having different and predetermined dissolution rates and times.

The smaller size of each particle, provided by microparticulation, also contributes to the better control of the release of 5-isosorbide mononitrate, since it reduces the influence of factors such as quality and periodicity of food ingestion and the activity periods and body rest during the day.

The compositions of the present invention overcome the problems of the prior art formulations, providing a product in which both drugs are formulated in a single dosage unit, reducing the risk of lack of use of one of the drugs prescribed, or inadequate use of the combination, while it provides a product with lower adverse effects risk, better profile and dissolution control, lower interactions with food or physical activity and easy administration, even in patients with swallowing difficulty.

The oral pharmaceutical compositions in which 5-isosorbide mononitrate and acetylsalicylic acid are formulated in the same unit dosage, through encapsulation or agglomeration, so as to maintain the integrity and individuality of the controlled-release of 5-isosorbide mononitrate particles, and the various particles that comprise the combination may be easily dispersed at the time of the administration or before it.

The process for the preparation of the compositions of the present invention includes the following steps:
a) mixing and homogenization of 5-isosorbide mononitrate controlled-release particles with acetylsalicylic acid particles and pharmaceutical excipients,
b) distribution of the mixture into oral dosage units, in which the individuality and physical integrity of the 5-isosorbide mononitrate particles are preserved (Fig. A).

According to the present invention, the 5-isosorbide mononitrate particles should be mixed with those of acetylsalicylic acid in a 1:6 to 3:1 ratio in terms of amounts of active compounds.

Controlled-release 5-isosorbide mononitrate particles can be obtained by a method selected from: inclusion of the drug in a thermoplastic matrix; application of the drug on an inert core and subsequent coating with one or more layers of polymeric and/or plastic materials; granulation of the drug together with polymeric hydrophobic or hydrophilic materials; coating crystals or granules with one or more layers of polymeric and/or plastic materials; inclusion of the drugs in micelles or liposomes; inclusion of the drug in release systems based in osmotic pressure; joined compression with polymeric hydrophobic or hydrophilic materials.

Acetylsalicylic acid may be present in the form of either multiple particles formulations similar to those described for 5-isosorbide mononitrate or regular-release formulations, separately, as granules or also as coated crystals.

The mixture of particles that contain 5-isosorbide mononitrate and acetylsalicylic acid should be preferably prepared together with conventional excipients, such as talc, Aerosil or magnesium stearate.

The doses of active compounds should be adequate to the therapy/prophylaxis of cardiovascular diseases and/or their signs/symptoms, such as angina pectoris, myocardial infarction or other vascular cardiac, cerebral or peripheral diseases.

The amount of 5-isosorbide mononitrate in each dosage unit is preferably of 25 to 100 milligrams, whereas the amount of acetylsalicylic acid is preferably 30 to 150 milligrams.

The dissolution rate of the slow-release 5-isosorbide mononitrate particles should provide effective plasma levels of the drug, preferably for a 24-hour period after the administration, more specifically for a 16- to 20-hour period after the administration. Therefore, the dissolution percentage profile of 5-isosorbide mononitrate in "in vitro" tests, according to the "paddle method - USP XXI", should preferably be: 15-45% after 1 hour; 30-60% after 2 hours; 45-75% in 4 hours; 55-85% in 6 hours; 65-95% in 8 hours; >70% in 12 hours.

The individual size of the particles of active ingredients should be small, to provide a more uniform flow of the drugs along the gastrointestinal tract, and cause a lower concentration of the drugs at the contact sites with the mucosa. The longer axis dimension should be preferably below 2 millimeters, more preferably below 1 millimeter.

### Example 1:

300 g of spherical sugar 0.4 to 0.5-mm diameter cores were coated with 500 g of 5-isosorbide mononitrate (dissolved in a mixture of 1.5 L of ethanol and 15 g of hydroxypropylmethylcellulose), followed by an intermediate layer of 50 g of polyvinylpyrrolidone (dissolved in 1 L of deionized water) in a fluidized bed equipment.

After drying to a water content lower than 1%, the granules were coated in the fluidized bed equipment, with an ethanol solution of 8% hydroxypropylmethylcellulose phthalate and 2% triethyl citrate. During the coating process, the thickness of this third layer was controlled so as to allow an adjustment of the dissolution profile of the isosorbide mononitrate. Therefore, granule portions with different layer thickness were removed during the process.

### Example 2:

A mixture of 500 g of 5-isosorbide mononitrate and 300 g powdered sugar was placed in a rotating granulator. Spherical granules were obtained by adding a glucose solution. The granules were classified in a diameter range of 0.6-0.7 mm and, afterwards, dried to a moisture contents lower than 1%. The resulting granules were then coated in a fluidized bed equipment with an intermediate layer of 50 g of hydroxypropylmethylcellulose (dissolved in 1.5 L of ethanol), followed by a layer of Eudragit L 100 of variable thickness, to adjust the dissolution profile of 5-isosorbide mononitrate.

### Example 3:

To a dry mixture of 500 g of 5-isosorbide mononitrate, 150 g of Eudragit RS 100 and 5 g of magnesium stearate, isopropyl alcohol was added until a mass with suitable plasticity to granulation was formed (approximately 200 ml). The damp mixture was then granulated through a pierced mesh, with 1-mm diameter holes. After drying in incubator at 50°C up to a moisture lower than 2%, the granules were reclassified using a 1-mm diameter holes mesh.

### Example 4:

A rotating granulator equipped with a heating device and granulating shaft was loaded with 500 g of 5-isosorbide mononitrate and 400 g of hard fat. The ingredients were heated and mixed at a rotation speed of 150 rpm until granules were formed, which were classified in a diameter range of 0.8 to 1 mm.

### Example 5:

In a rotating granulator, a mixture of 500 g of acetylsalicylic acid and 200 g of powdered sugar was placed. Spherical granules were obtained by adding a glucose solution. The granules were classified in a diameter range of 0.6-0.7 mm and, afterwards, dried up to a moisture contents lower than 1%. The resulting granules were then coated in a fluidized bed equipment with an enteric layer of 50 g of hydroxypropylmethylcellulose phthalate and 0.25 g of triethyl citrate (dissolved in 1.5 L of ethanol).

### Example 6:

A dry mixture of 500 g of acetylsalicylic acid, 15 g of polyvinylpirrolidone and 5 g of magnesium stearate, was added with ethanol until a mass with proper plasticity to granulation was formed (approximately 200 ml). The damp mixture was then granulated through a pierced mesh, with 1-mm diameter holes. After drying in incubator at 50°C up to a moisture lower than 2%, the granules were reclassified using a 1-mm diameter holes mesh.

### Example 7:

130 g of the 5-isosorbide mononitrate granules obtained in reference example 1 were mixed with 150 g of the acetylsalicylic acid granules obtained in the reference example 5, and 1 g of talc. Hard gelatin #1 capsules were filled with about 280 mg of the mixture, corresponding to 50 mg of isosorbide mononitrate and 100 mg of acetylsalicylic acid. The dissolution profiles of isosorbide mononitrate and acetylsalicylic acid were the same as those of the granules tested separately.

### Example 8:

65 g of the granules of 5-isosorbide mononitrate obtained in reference example 1 were mixed with 130 g of the acetylsalicylic acid granules obtained in reference example 5, and 1 g of talc. Hard gelatin #2 capsules were filled with about 280 mg of the mixture, corresponding to 50 mg of 5-isosorbide mononitrate and 100 mg of acetylsalicylic acid. The dissolution profiles of isosorbide mononitrate and acetylsalicylic acid were the same as those of the granules tested separately.

### Example 9:

200 g of acetylsalicylic acid coated crystals (acetylsalicylic acid 75%; coating 25%) were mixed with 1 g of magnesium stearate and 4 g of talc. Hard gelatin #1 capsules were loaded with 205 milligrams of this mixture, corresponding to 150 mg of acetylsalicylic acid. 160 mg of a mixture of 4 g of magnesium stearate and 156 g of the granules obtained in reference example 2 were added to the semi-filled capsules, corresponding to 60 mg of 5-isosorbide mononitrate. The dissolution profile of isosorbide mononitrate was the same as that of the granules tested separately. After 30 minutes, acetylsalicylic acid was completely dissolved.

### Example 10:

100 g of acetylsalicylic acid coated crystals (acetylsalicylic acid 75%; coating 25%) were mixed with 1 g of magnesium stearate and 4 g of talc. Hard gelatin #2 capsules were loaded with 105 milligrams of this mixture, corresponding to 75 mg of acetylsalicylic acid. 100 mg of a mixture of 4 g of magnesium stearate, 6 g of talc and 90 g of the granules obtained in reference example 4 were added to the semi-filled capsules, corresponding to 50 mg of 5-isosorbide mononitrate. The dissolution profile of isosorbide mononitrate was the same as that of the granules tested separately. After 30 minutes, acetylsalicylic acid was completely dissolved.

### Example 11:

130 g of the 5-isosorbide mononitrate granules obtained in reference example 1 were mixed with 104 g of the acetylsalicylic acid granules obtained in example 6, 30 g of glycolate sodium starch, 70 g of sorbitol and 2 g of sodium stearate. Tablets with 340 mg of the mixture were obtained, corresponding to 50 mg of 5-isosorbide mononitrate and 100 mg of acetylsalicylic acid. The dissolution profile of the isosorbide mononitrate of the tablets was substantially the same as that of the granules tested separately. The disintegration time of the tablets was lower than 15 seconds. After 30 minutes, the acetylsalicylic acid was completely dissolved.

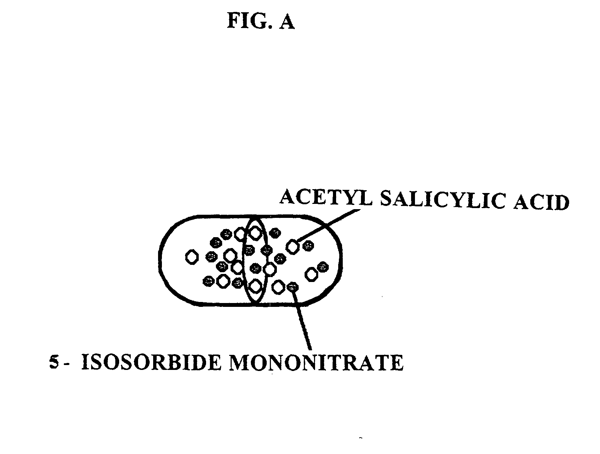

## Claims

1. An oral pharmaceutical composition comprising controlled-release granules of 5-isosorbide mononitrate in combination with acetylsalicylic acid particles, optionally in admixture with suitable excipients, obtained by a process comprising the step of:
a) mixing and homogenization of 5-isosorbide mononitrate controlled release granules with acetylsalicylic acid particles and pharmaceutical excipients;
b) distribution of the mixture into oral dosage units in which the individuality and physical integrity of the 5-isosorbide mononitrate granules are preserved.

2. A composition according to claim 1, wherein the size of the controlled-release 5-isosorbide mononitrate granules is lower than 2 millimeters.

3. A composition according to claim 2, wherein the longer axis size of the controlled-release 5-isosorbide mononitrate granules is lower than 1 millimeter.

4. A composition according to any one of claims 1-3, wherein the controlled-release 5-isosorbide mononitrate granules have the following percentage dissolution profile of 5-isosorbide mononitrate in "in vitro" tests, according to the "paddle method - USP XXI": 15-45% after 1 hour; 30-60% after 2 hours; 45-75% in 4 hours; 55-85% in 6 hours; 65-95% in 8 hours; higher than 70% in 12 hour.

5. A composition according to any one of claims 1-4, wherein the acetylsalicylic acid particles are controlled-release particles, granules, coated crystals or regular-release particles.

6. A composition according to any one of claims 1-5, wherein the weight ratio of 5-isosorbide mononitrate to acetylsalicylic acid ranges from 1:6 to 3:1.

7. A composition according to any one of claims 1-6, in the form of a single dosage unit.

8. A composition according to claim 7, in the form of capsules or tablets.

9. A composition according to any of the preceding claims, wherein the 5-isosorbide mononitrate granules are obtained by a method selected from: inclusion of the drug in thermoplastic matrix; application of the drug on an inert core and subsequent coating with one or more layers of polymeric and/or plastic materials; granulation of the drug together with polymeric hydrophobic or hydrophilic material; coating of crystals or granules with one or more layers of polymeric and/or plastic materials; inclusion of the drug in micelles or liposomes; inclusion of the drug in release systems based on osmotic pressure; joined compression with polymeric hydrophobic or hydrophilic materials.

## Patentansprüche

1. Orale pharmazeutische Zusammensetzung, die Körnchen von 5-Isosorbidmononitrat mit einer gesteuerten Freisetzung in Verbindung mit Acetylsalicylsäure-Partikeln, gegebenenfalls im Gemisch mit geeigneten Exzipienzien, umfasst und durch ein Verfahren erhalten wird, das die Schritte umfasst:
a) Mischen und Homogenisieren von 5-Isosorbidmononitrat-Körnchen mit gesteuerter Freisetzung mit Acetylsalicylsäure-Partikeln und pharmazeutischen Exzipienzien,
b) Verteilen des Gemisches in orale Dosiseinheiten, in denen die Individualität und physikalische Integrität der 5-Isosorbidmononitrat-Körnchen gewahrt bleiben.

2. Zusammensetzung nach Anspruch 1, wobei die Größe der 5-Isosorbidmononitrat-Körnchen mit gesteuerter Freisetzung geringer als 2 mm ist.

3. Zusammensetzung nach Anspruch 2, wobei die Größe der längeren Achse der 5-Isosorbidmononitrat-Körnchen mit gesteuerter Freisetzung weniger als 1 mm beträgt.

4. Zusammensetzung nach einem der Ansprüche 1 bis 3, wobei die 5-Isosorbidmononitrat-Körnchen mit gesteuerter Freisetzung das nachstehende prozentuale Auflösungsprofil von 5-Isosorbidmononitrat in "in vitro"-Tests gemäß dem "Paddle-Verfahren - USP XXI" aufweisen: 15-45% nach 1 Stunde, 30-60% nach 2 Stunden, 45-75% innerhalb von 4 Stunden, 55-85% innerhalb von 6 Stunden, 65-95% innerhalb von 8 Stunden, mehr als 70% innerhalb von 12 Stunden.

5. Zusammensetzung nach einem der Ansprüche 1 bis 4, wobei die Acetylsalicyclsäure-Partikel Partikel mit gesteuerter Freisetzung, Körnchen, beschichtete Kristalle oder Partikel mit einer normalen Freisetzung sind.

6. Zusammensetzung nach einem der Ansprüche 1 bis 5, wobei das Gewichtsverhältnis von 5-Isosorbidmononitrat zu Acetylsalicylsäure 1:6 bis 3:1 beträgt.

7. Zusammensetzung nach einem der Ansprüche 1 bis 6, die in Form einer einzelnen Dosiseinheit vorliegt. '

8. Zusammensetzung nach Anspruch 7, die in Form von Kapseln oder Tabletten vorliegt.

9. Zusammensetzung nach einem der vorhergehenden Ansprüche, wobei die 5-Isosorbidmononitrat-Körnchen durch ein Verfahren erhalten werden, ausgewählt aus:
Einschluss des Arzneimittels in eine thermoplastische Matrix, Auftragen des Arzneimittels auf einen inerten Kern und darauf folgendes Beschichten mit einer oder mehreren Schichten an polymeren und/oder plastischen Materialien, Granulieren des Arzneimittels zusammen mit polymerem hydrophobem oder hydrophilem Material, Beschichten von Kristallen oder Körnchen mit einer oder mehreren Schichten an polymeren und/oder plastischen Materialien, Einschluss des Arzneimittels in Micellen oder Liposomen, Einschluss des Arzneimittels in Freisetzungssysteme, die auf osmotischem Druck basieren, gemeinsames Verpressen mit polymeren hydrophoben oder hydrophilen Materialien.

## Revendications

1. Composition pharmaceutique orale comprenant des granulés à libération contrôlée de mononitrate de 5-isosorbide en combinaison avec des particules d'acide d'acétylsalicylique, éventuellement en mélange avec des excipients convenables, obtenue par un procédé comprenant l'étape de :
a) mélange et homogénéisation des granulés à libération contrôlée de mononitrate de 5-isosorbide avec les particules d'acide acétylsalicylique et les excipients pharmaceutiques ;
b) distribution du mélange dans des doses orales dans lesquelles l'individualité et l'intégrité physique des granulés de mononitrate de 5-isosorbide sont conservées.

2. Composition selon la revendication 1, dans laquelle la taille des granulés à libération contrôlée de mononitrate de 5-isosorbide est inférieure à 2 millimètres.

3. Composition selon la revendication 2, dans laquelle la taille de l'axe plus long des granulés à libération contrôlée de mononitrate de 5-isosorbide est inférieure à 1 millimètre.

4. Composition selon l'une quelconque des revendications 1 à 3, dans laquelle les granulés à libération contrôlée de mononitrate de 5-isosorbide ont le profil de pourcentage de dissolution suivant du mononitrate de 5-isosorbide dans des essais "in vitro", selon le "procédé à palette (paddle method) - USP XXI" : 15 à 45 % au bout de 1 heure ; 30 à 60 % au bout de 2 heures ; 45 à 75 % au bout de 4 heures ; 55 à 85 % au bout de 6 heures ; 65 à 95 % au bout de 8 heures ; supérieur à 70 % au bout de 12 heures.

5. Composition selon l'une quelconque des revendications 1 à 4, dans laquelle les particules d'acide acétylsalicylique sont des particules à libération contrôlée, des granulés, des cristaux enrobés ou des particules à libération régulière.

6. Composition selon l'une quelconque des revendications 1 à 5, dans laquelle le rapport pondéral du mononitrate de 5-isosorbide à l'acide acétylsalicylique s'échelonne de 1:6 à 3:1.

7. Composition selon l'une quelconque des revendications 1 à 6, sous forme de dose unique.

8. Composition selon la revendication 7, sous forme de capsules ou de comprimés.

9. Composition selon l'une quelconque des revendications précédentes, dans laquelle les granulés de mononitrate de 5-isosorbide sont obtenus par un procédé choisi parmi : l'incorporation du médicament dans une matrice thermoplastique ; l'application du médicament sur une noyau inerte et l'enrobage ultérieur avec une ou plusieurs couches de matériaux polymères et/ou plastiques ; la granulation du médicament avec un matériau polymère hydrophobe ou hydrophile ; l'enrobage de cristaux ou de granulés avec une ou plusieurs couches de matériaux polymères et/ou plastiques ; l'incorporation du médicament dans des micelles ou des liposomes ; l'incorporation du médicament dans des systèmes de libération basés sur la pression osmotique ; la compression conjointe avec des matériaux polymères hydrophobes ou hydrophiles.
